(19) Europäisches Patentamt
European Patent Office
Office européen des brevets

(11) Publication number: **0 469 632 A1**

## EUROPEAN PATENT APPLICATION

(21) Application number: **91113061.5**

(22) Date of filing: **02.08.91**

(51) Int. Cl.⁵: **C12Q 1/18**, C12Q 1/70,
A61K 35/12, G01N 33/569

(30) Priority: **03.08.90 US 562746**

(43) Date of publication of application:
**05.02.92 Bulletin 92/06**

(84) Designated Contracting States:
**AT BE CH DE DK ES FR GB GR IT LI LU NL SE**

(71) Applicant: **SYSTEMIX, INC.**
**3400 West Bayshore Road**
**Palo Alto, California 94303(US)**

(72) Inventor: **McCune, Joseph M.**
**51 Clipper**
**San Francisco, California 94114(US)**
Inventor: **Mocarski, Edward S., Jr.**
**141 Erica Way**
**Portola Valley, California 94028(US)**

(74) Representative: **Glawe, Delfs, Moll & Partner**
**Patentanwälte**
**Liebherrstrasse 20**
**W-8000 München 26(DE)**

(54) Modulated xenogeneic organ systems in a immunocompromised host.

(57) Xenogeneic tissue is introduced into an immunocompromised host for interacting with agents and using such interaction for evaluating efficacy of drugs and vaccines, evaluating the effect of the various agents on specific tissues and the like. Particularly, drugs can be evaluated for their efficacy against a wide variety of pathogens which infect xenogeneic tissue, and agents can be evaluated for their effect on the xenogeneic immune system.

CROSS-REFERENCE TO RELATED APPLICATIONS

This application is a continuation-in-part of application serial number 562,746, filed August 3, 1990, which is a continuation-in-part of application serial number 518,748, filed May 3, 1990, which is a continuation-in-part of application serial number 462,823, filed January 10, 1990, which is a continuation-in-part of application serial number 287,075, filed December 20, 1988, which is a continuation-in-part of application serial number 137,173, filed December 23, 1987.

INTRODUCTION

Technical Field

The field of this invention is immunocompromised mammals compromising xenogeneic tissue and their use with non-retroviral pathogenic agents.

Background

The field of medicine for the treatment of humans has depended for the most part on animal models where the tissue involved was the animal tissue. These models have frequently involved pathogenic agents which had a broad host range including human and other mammalian hosts, pathogenic agents which were capable of causing disease in the animal model which were analogous to a pathogenic agent capable of causing disease in humans, or caused diseases in animals which were etiologically and symptomatically analogous to the disease in humans, and the like. There are many shortcomings with these techniques. Since the cells and tissue which are involved with the pathogens are not human tissue, and they may differ in a wide variety of ways in their response from the response of human tissue, there is always the uncertainty as to whether the animal cells provide a reasonable predictor of what may be anticipated in a human. Also, with many genetic diseases, the same lesion in an animal may have a significantly different effect in humans.

There is also interest in being able to understand how human cells respond to various stimuli, how different types of cells interact, within the bone marrow, such as stromal endothelium cells and bone marrow, lymphoid and myeloid cells, and the like.

Having viable human tissue in an animal model provides for numerous advantages. One can investigate the effect of various agents on the tissue at various stages in the development of the cells and the tissue. In addition, one can investigate the interaction of cells and secreted agents on tissue, as well as the communication between various tissues and the feedback and regulation of the metabolism of the cells. Importantly, one may introduce various agents, both natural and synthetic, into the host to determine the effect of the agents on the tissue, its metabolism, proliferation and differentiation. In addition, one may use combinations of the reagents, such as pathogens and drugs, to determine the effect of the pathogen on the tissue and the effect of the drug on the pathogen and the tissue.

It is therefore of substantial interest to develop and provide mammalian models comprising human tissue, which remains viable for extended periods of time, is responsive to various agents and permits the use of techniques which allow for the investigation of the changes in the tissue, the etiology of disease and the effect of agents on pathogens and tissue for prophylaxis and therapy. Thus, there is a need for an animal model which allows for study of both physiological and pathophysiological conditions and agents which can modulate such conditions.

Relevant Literature

References concerned with immunoincompetent hosts, particularly CID or SCID hosts, include McGuire et al., Clin. Immunol. and Immunopath. (1975) 3:555-566; Perryman and Torbeck, J. Am. Vet. Med. Assoc. - (1980) 176:1250-1251; Shultz and Sidman, Genetically-determined Murine Models of Immunodeficiency, The Jackson Laboratory, Bar Harbor, ME; Bosma et al., Nature (1983) 301:527-530; Custer et al., Amer. J. Path. (1985) 120:464-477; Dorshkind et al., J. of Immunol. (1985) 134:3798-3801; Kerghtley et al., Lancet, November 1, 1975, 850-853; Touraine, Immunological Rev. (1983) 71:103-121; and Fulop and Phillyes, J. of Immunology (1986) 136:4438-4443.

References concerned with xenogeneic cells growing within live hosts include Krueger et al., Proc.Natl. Acad. Sci. USA (1983) 80:1650-1654; Krueger and Shelby, J. Inv. Dermatol. (1981) 76:506-510; Ware et al., J. Immunol Meth. (1985) 85:353-361; Ford et al., Nature (1956) 177:452-454; Povlsen, et al., Nature (1974) 248:247-249; Mannhardt et al., Thymus (1982) 4:209-220; Schulte-Wisserman et al., Scand. J. Immunol. - (1978) 8:387-396. Please specifically note, McCune et al., Science (1988) 241:1632-1639 and the comment therein; Yancopoulos and Alt, Ibid (1988) 241:1581-1583, and references cited therein.

See also EPA 0 322 240.

## SUMMARY OF THE INVENTION

Methods, non-human mammals and organs are provided for initiating cellular and/or tissue response under conditions where the response may be evaluated to determine the efficacy of the agents and/or conditions, both prophylactic and therapeutic, to investigate cellular and tissue response to various agents and conditions. The non-human mammals are characterized by being immunocompromised, having a viable, xenogeneic organ or tissue capable of functioning at least in part as the organ or tissue from which it is derived, which tissue is vascularized, and, as appropriate, connected with lymphatic vessels.

## DESCRIPTION OF THE SPECIFIC EMBODIMENTS

Methods, compositions and organisms are provided where the organisms are characterized by having solid xenogeneic tissue, normally human tissue, in a non-human viable host where the solid tissue is viable, functional and able to respond to various stimuli and allow for the analysis of the response, and the investigation of various physiological processes.

The solid implants are able to function for long periods of time, usually in excess of two weeks, more usually in excess of four weeks, and depending upon the particular implant, the site of the implant, and the tissue, which may include a variety of cells, including hematopoietic, stromal, lung, fibroblasts, epithelium, endothelium, neurons, stem cells, or other cells associated with particular solid organs, such as bone marrow, pancreas, appendix, tonsil, gut, lung, GALT (gut-associated lymphoid tissue), MALT (mucosa-associated lymphoid tissue), tongue, mucosal tissue, adrenal gland, thymus, liver, central nervous system tissue, spinal cord, thyroid, pituitary gland, hypothalamus, bone, including osteoclasts and osteoblasts, muscle, including myoblasts, myocytes, neuronal tissue and the like.

Among the organs and cells associated with hematopoiesis are stem cells (precursor cells for the various human hematopoietic lineages, e.g., lymphoid, myelomonocytic and erythroid) and cells providing a processing organ, such as the thymus, bone marrow, spleen, lymph node, tonsils, appendix and skin. Cells of interest associated with the hematopoietic system include stem cells, stromal cells, monocytes, macrophages, B-cells, T-cells, neutrophils, erythrocytes, eosinophils, megakaryocytes, platelets, dendritic cells, natural killer cells, cytotoxic T-lymphocytes (CTLs), tumor-infiltrating lymphocytes (TILs), helper cells (CD4), suppressor cells (CD8), lymphocyte-activated killer cells (LAKs), antibody dependent cytotoxic cells (ADCC), and the like.

A wide variety of tissues may find use and be able to grow in the subject host. In addition, the tissues may be subject to induction of disease or may be available in their diseased form. The following table provides a list of tissues of interest with various pathologic conditions associated with such tissue.

### TABLE 1

| Tissue | Pathologic Condition |
|---|---|
| Connective tissue | Rheumatic fever, systemic lupus erythematosis, rheumatoid arthritis, gout |
| Brain (nerve) tissue | Paralysis, Alzheimer's disease, multiple sclerosis, Lou Gehrig's disease, glycogenoses, encephalitis |

| | |
|---|---|
| Synovial tissue | Rheumatoid arthritis |
| Respiratory tissue | Emphysema, edema, Goodpasture's syndrome, sarcoidosis |
| Liver | Cirrhosis, Wilson's disease, Pompe's disease, Forke's disease, hepatitis, primary biliary cirrhosis, $alpha_1$-antitrypsin disease, hemachromatosis, glycogenoses |
| Kidney | Glomerulonephritis, pyelonephritis, lupus nephritis, tubular acidosis, ketoacidosis, renal failure, cystinuria |
| Thyroid | Hypothyroidism, hyperthyroidism, Grave's disease, goiter, Hashimoto's disease |
| Colon | Peptic ulcer, stomach or duodenum, diverticulosis, inflammatory bowel disease |
| Skin | Ulcer, gangrene, psoriasis, erythroderma syndrome, pemphigus pemphigoid |
| Lymph node | Lymphadenopathy, lymphoma |
| Blood vessels | Vasculitis, Wegener's granulomatosis, giant cell vasculitis, polyarteritis nodosa |
| Pancreas | Diabetes, pancreatitis |
| Breast | Carcinoma 1° or 2° |
| Pineal Gland | Circadian rhythms |
| Prostate | Hypertrophy, CA |
| Testes | Hypogonadism |
| Ovary | Turner's syndrome, Stein-Leventhal syndrome, cyst |
| Reticuloendothelium | Gaucher's disease, Letterer-Siwe disease |
| Stomach | Peptic ulcer, gastritis, pernicious anemia, ulcerative colitis |
| Salivary gland | Sjögren's disease |
| Muscle | Myasthenia gravis, polymyositis, muscular dystrophy |
| Parathyroid | Primary hypoparathyroidism |
| Adrenal gland | Addison's disease, Cushing's disease, Conn's disease |

In other situations, it may be of interest to study the pathogenesis of various infectious agents and/or the effect of various drugs or treatments on the induction or progress of the disease. The following table provides a list of infectious agents of interest and tissue which may find application in the study of these various infectious agents. The table is not intended to be exhaustive, and in some instances, one tissue will be preferred over another tissue in relation to a particular agent. Furthermore, there may be situations where a tissue which is not listed is employed, because of availability of such infected tissue, level of viability of the tissue in the host, and the like.

TABLE 2

| Infectious Agents | Human Tissue |
|---|---|
| Gram-negative bacteria | Lung, kidney, myocardium, capillaries (endothelium) |
| Pneumococcus | Lung (alveolar walls), trachea |
| Staphylococcus | Integument, long bones, endocardium, myocardium, liver, spleen, brain |
| Streptococcus | Pharynx, mucosal tissue, tonsil, skin, sinus |
| Meningococcus | Pharynx, mucosal tissue |
| Gonococcus | Urethra, vulva |
| Enteric gram-negative bacilli | Lung |
| E. coli | Urethra, appendix, mucosal tissue |
| Klebsiella-Enterobacter-Serratia | Lung, pharynx, urethra |
| Proteus | Skin, bone, urethra |
| Pseudomonas | Skin, urethra, lung |
| Salmonella | Gut, spleen |
| Shigella | Gut |
| Hemophilus | Mucosal tissue, pharynx, tonsil |
| Yersinia | Lymph node |
| Listeria | Skin, liver, spleen, adrenal gland |
| Corynebacterium | Membrane, mucosal tissue, tonsil, pharynx |

| | |
|---|---|
| Vibrio (cholera) | Mucosal tissue, gut |
| Clostridium (tetanus) | Skin |
| Clostridium (botulism) | Gut, mucosal tissue |
| Mycobacterium (tuberculosis) | Lung, lymph node |
| Mycobacterium (leprosy) | Skin, nerves, respiratory mucosa |
| Treponema (syphilis) | Mucosal tissue |
| Histoplasma | Skin, lung |
| Candida | Mucosal tissue |
| Mycoplasma | Bronchia, pharynx, trachea, mucosal tissue |
| Chlamydia | Conjunctiva, cornea |
| Viruses | |
| Enteric viruses: Coxsackie, Echo, Reo | Muscle, viscera, kidney, mucosal tissue |
| Respiratory viruses: Rhino, adeno, RSV, parainfluenza, corona | Mucosal tissue, bronchia, pharynx |
| Influenza virus | Respiratory tissue, mucosal tissue |
| Picorna (poliomyelitis) | Pharynx, gut |
| Rhabdovirus (rabies) | Muscle, nerve tissue, brain |
| Slow virus | Brain |
| Measles (rubeola) | Conjunctiva, mucosa |
| Rubella | Pharynx, mucosal tissue |
| Smallpox | Pharynx, mucosal tissue |
| Herpes: Zoster | Pharynx, mucosal tissue |
| Simplex I and II | Respiratory tissue, conjunctiva, labia, ganglia, brain |
| EBV | Lymph node, thymus, spleen |
| Mumps (paramyxovirus) | Lymph node, mucosal tissue, meninges |
| Cytomegalovirus | Lymph node, thymus, bone marrow, epithelial tissue |

Among classes of viruses which may be studied are picornaviridae, enteroviruses, rhinoviruses, hepatitis viruses, caliciviridae, Norwalk group, togaviridae, flaviviridae, alphaviruses, rubella, coronaviridae, rhabdoviridae, filoviridae, paramyxoviridae, parainfluenza viruses, mumps virus, measles virus, respiratory syncytial virus, orthomyxoviridae, bunyaviridae, arenaviridae, reoviridae, rotaviruses, lentivirus, orbiviruses, retroviridae, T-cell leukemia viruses, lentiviruses, papovaviridae, polyoma viruses, papilloma viruses, ad-

6

EP 0 469 632 A1

enoviridae, parvoviridae, herpesviridae, herpes simplex viruses, Epstein-Barr virus, cytomegalovirus, Varicella-Zoster virus, poxviridae, hepadnaviridae, hepatitis viruses A, B and C, and such additional viruses or strains which particularly have human tropism. The retroviridae are not included.

In addition, one may choose to use normal tissue which has been subjected to a treatment which allows the normal tissue to mimic human tissue involved with a pathological condition. In this manner, one may provide for various models which would permit the investigation of pathological conditions in tissue. Thus, one may induce hypoxia, lesions, ulcerations, cells which provide cell mediated degeneration, e.g., T-cells, neutrophils, killer cells, etc., genetic diseases by transfection with genetically modified viruses to provide for or inhibit a genetic capability, administrations of toxins, inducing stress, and the like. The list is not intended to be exhaustive, but indicates a variety of tissues which may be used, where pathologic conditions may be induced by various means.

TABLE 3

| Tissue | Pathologic Condition |
|---|---|
| Heart | Infarction, hypertension, stenosis, ischemia |
| Vascular system | Vessel wall abnormalities, atherosclerosis, thromboembolism |
| Lymphatic system | Homing (inflammatory), reperfusion injury |
| Pancreas | Diabetes |
| Synovia | Arthritis |
| Lung | Asthma, mineral dust, ARDS |
| Kidney | Glomerulonephritis |
| Gut | Homing (inflammatory), ulcer, nutrient absorption |
| Liver | Bilirubin turnover, cirrhosis, hepatitis (acute toxic exposure) |
| Bone marrow | Anemia |
| Brain and CNS | Multiple sclerosis, myasthenia gravis, Alzheimer's disease |
| Muscle | Muscular dystrophy |
| Bone | Osteoporosis |
| Joint | Tendinitis, bursitis, arthritis, ankylosing spondylitis |

In many instances, it may be desirable to have more than one type of tissue, for example, where one wishes to study the effect of an agent on the immune system or a subset of the immune system, while at the same time studying its efficacy against a pathogen. Thus, in addition to implants or grafts not associated with lymphoid tissue, one may also wish to introduce lymphoid tissue to provide for a response, either cellular or humoral. Hematopoietic stem cells may be introduced into the host in conjunction with embryonic yolk sac, fetal liver, thymus, spleen or lymph node tissue, fetal or adult bone marrow tissue, pancreatic tissue, appendix tissue, tonsil tissue and the like. Other stem cell systems may be employed. In other than the hematopoietic system, combinations may include stem cells of the central or peripheral nervous system, such as neuronal or matrix cells of the fetal central nervous system, spinal cord neurons and the spinal cord, organs of the endocrine system, such as beta-cells and other cells of the islets and pancreas, the adrenal gland, the thyroid gland, and the hypothalamo-pituitary axis. The stem cells will be introduced in combination with the appropriate organs in which each of them is induced to differentiate and to become functional.

In some instances, it will be desirable to enhance the proportion of human cells in the peripheral blood, providing for human cells being at least 0.1 percent, preferably at least about one percent, more preferably at least about five percent of the total circulating blood cells. The host bones, in whole or in part, may be irradiated or treated with cytotoxic drugs at a level which will at least partially ablate the endogenous hematopoietic cells. With each type of host, the particular level of treatment may be selected by screening for the level of hematopoietic cells remaining and the proportionate number of viable hosts. Instead of x-irradiation, various cytotoxic drugs may be employed, such as immunotoxins. The bone marrow, as bone or dispersed, will usually be fetal bone marrow and may be employed as chunks, slices, fragments or the like, the size of the bone depending on the size of the host. For a mouse, the bone will generally be less than about 2 cm, more usually less than about 1 cm, providing a total volume of bone of from about 40 to 1200 mm$^3$. The site of introduction of the bone may be intraperitoneal, subcutaneous, mammary fat pad or the like.

Immunocompromised mammalian hosts having the desired immune incapacity exist or can be created. The significant factor is that the immunocompromised host is incapable naturally or in conjunction with the introduced organs to be able to mount an immune response against the xenogeneic tissue or cells. Therefore, it is not sufficient that a host be immunocompromised, but the host may not be able to mount an immune response after grafting, as evidenced by the inability to produce competent B-cells, particularly plasma cells, and/or T-cells, particularly $CD4^+$ and/or $CD8^+$ T-cells. Hosts which are presently available include hosts which have a severe combined immunodeficiency, known as scid/scid.

For pathogens other than retroviridae, tissue capable of being infected is transplanted into an appropriate site in the host, usually mouse, and allowed to form whatever connections are appropriate, such as vascularization or connection with lymphatics, or other organs or vessels in the host. Once the tissue(s) and, as appropriate, circulating cells have become established, the tissue may then be subjected to the agent for investigation or product production, depending upon the nature of the tissue(s), the agent, the purpose for which the agent is used, convenience, and the like. The agent may be introduced by injection, intravascularly, intraperitoneally, directly into the tissue, topically, intranasally by an aerosol, or the like.

Depending upon the particular agent and investigation, various bioassays may be employed. Where one is interested in replication, such as in viremia, one may detect various proteins produced by the virus or nucleic acid associated with the virus, at the tissue site, in the peripheral blood, or other site of interest. Various assays exist for identifying specific DNA, virus particles, specific proteins, or the like, where these assays find extensive exemplification in the literature and need not be detailed here.

As paradigmatic of infectious agents, particularly viruses other than retroviridae, is the herpes virus, cytomegalovirus. While the herpes viruses are DNA viruses, they are also exemplary of many RNA viruses, other than the retroviruses. CMV is a complex virus having a genome of about 240 kbp and is host specific, as well as having some tissue preference. In the SCID-hu mouse, the virus is found to infect a joint engraftment of human fetal thymus and liver, colon, bone and skin. The infection is found to be stable for extended periods of time and the viremia is responsive to agents toxic for CMV, such as ganciclovir.

The following examples are offered by way of illustration and not by way of limitation.

EXPERIMENTAL

Methods

1. Mice.

CB-17 scid/scid mice were obtained from Dr. Leonard D. Shultz of The Jackson Laboratory, Bar Harbor, ME. The mice are housed in standard isolator cages within a routine animal holding facility. Under these conditions, they have a life span that is considerably shorter than that of other inbred immunocompetent strains (e.g., 1-2 years vs. 3-4 years). The cause of death is normally related to opportunistic infection (most often by Pneumocystis carinii). Protocols to prevent such infections by administering prophylactic antibiotics (trimethoprim/sulfamethoxasole) to the mice are being employed (see above) using as guidelines protocols developed for the prophylaxis of patients with AIDS or ARC. In all other respects (e.g., bedding, food, daily light cycles, etc.), the mice are handled as per routine animal holding facility protocols.

2. Collection and preparation of human fetal tissues.

The information that is known about the patient includes the approximate (or where known, the actual) gestational age of the fetus and the given reason for the abortion; in the latter case, also known are the details of any genetic or morphologic anomaly discovered by amniotic fluid analysis and/or ultrasonography (e.g., chromosomal defects, anencephaly, hydrops, etc.). In initial experiments, tissue from fetuses that are apparently normal were used; in later experiments, specifically constructed situations were created in which tissues from fetuses with genetic anomalies were tested.

The tissues are obtained directly in the operating room as fetal parts after elective or medically-indicated abortion (with gestational ages ranging from 7-22 weeks). Without maintaining strict sterility, these parts are taken immediately to a gross dissection room. The desired tissues are identified, dissected out, placed into RPMI 1640 medium with 10% fetal calf serum, and transported directly to another lab. In those situations in which "whole organ" transplants (i.e., tissue inclusive of both the stromal elements and of the hematopoietic elements therein) are being performed, the relevant organs are cut into sections that are approximately 1 mm by 4 mm. (A piece of tissue of this size fits easily into the 19-gauge trocar that is used for implantation.) In those situations in which dispersed cells are to be injected, the relevant organs are teased apart to yield viable cells in suspension. In the case of fetal liver, the suspension cells are enriched for hematopoietic precursors by ficoll-hypaque density gradient centrifugation; the interface layer containing the desired cells is then washed 3x, counted, and brought to approximately $10^8$ cells/ml. For subfractionation of hematopoietic precursor cells, fetal liver cells isolated on ficoll-hypaque gradients are subsequently stained with monoclonal antibodies against relevant cell surface markers and isolated by techniques including negative selection with magnetic beads and positive selection on the fluorescence activated cell sorter (FACS).

To mark the genetic origin of the donor fetal tissue, HLA histotyping is performed on fetal thymocytes using monoclonal antibodies (see below) that recognize common HLA alleles. In this manner, prior to implantation, a histotyped "fingerprint" is obtained by which all subsequent progeny of the introduced stem cells may be specifically followed in the SCID-hu mouse.

Tissue is normally introduced in as fresh a state as possible. Therefore, the tissue collection, preparation, histotyping, and implantation are done all on the same day. Frozen tissue, however, appears to work well in the case of fetal liver cells and fetal thymus tissue. Therefore, aliquots of remaining tissue from each specimen are frozen down at the end of the day 10%DMSO/50%FCS using standard procedures and then catalogued in a liquid nitrogen storage freezer.

## 3. Transplantation of human tissue into SCID mice.

Mice have generally been used at 4-8 weeks of age, either without pretreatment or after pretreatment with either (a) anti-asialoglycoprotein GM1 or (b) fractionated courses of irradiation (175 rads weekly x 4 weeks). The latter two pretreatment regimens have been designed to test the possibility that removal of endogenous natural killer cell activity in the SCID may permit better constitution with xenogeneic tissue.

Tissue has been introduced by a number of routes: intravenously, beneath the kidney capsule, intrasplenically, intraperitoneally or subcutaneously. In the latter three cases, the mice are first anesthetized with halothane. A 1 cm incision is made to expose either the kidney or the spleen and a 19 g trocar is used to introduce human tissue beneath the capsule of either organ. Thereafter, the incision is approximated with surgical sutures. For intravenous injections, a 30 g needle is used to introduce suspension cells into the retro-orbital venous plexus. Of these routes, implantation of tissue into the left kidney capsule affords several distinct advantages: first, it is easily accessible; secondly, the kidney may be exposed repeatedly to observe the growth of the transplanted tissue and to remove biopsy specimens from it for histologic analysis.

## 4. Analysis of SCID-hu mice.

### A. Immunophenotyping.

A number of monoclonal antibodies against various cell surface markers of human lymphocytes have been prepared for the analysis of cells found within SCID-hu mice. These include markers of mature human peripheral blood T-cells (OKT4, OKT8, OKT3, OKT11), markers of human peripheral blood B-cells (CD19, CD20, anti-IgM, anti-IgG, anti-light chain), and markers of the human HLA complex (including both polymorphic and monomorphic determinants of Class I antigens). These murine antibodies have been used in immunofluorescence assays, either as primary antibodies that are secondarily reacted with fluoresceinated (FITC) anti-mouse antibody, or as biotinylated antibodies that are secondarily reacted with avidin-FITC. In some instances, antibodies that are directly fluoresceinated or coupled with phycobiliproteins have also been used. After transplantation, mice are bled weekly by tail vein incision or retroorbital bleed; approximately 100 $\mu$l of whole blood is obtained (usually containing 1-2 x $10^5$ cells). Nucleated cells are enriched by precipitation of red blood cells with dextran sulfate, or by lysis of red blood cells with hypotonic shock, washed free of platelets, and then stained with the monoclonal antibodies within 96-well microtiter plates. After the assay is complete, a visual inspection for positive cells (that is, those with unambiguous surface fluorescence) is made under the immunofluorescence microscope. If human cells represent greater than 1% of the total nucleated cells in the periphery, they may be readily detected by this method and also quantitated with statistical significance with a multiple parameter FACS. Accordingly, if the samples are positive by direct inspection, they are then analyzed by FACS. In this analysis, the cells are fixed first with 1% formalin and then incubated for 10 min with 1 $\mu$g/ml propidium iodide (PI) (which is taken up by nucleated cells). In subsequent FACS analyses, cells are first gated for PI (that is, they are nucleated) and then assessed for staining with FITC. In this manner, the percentage of FITC-positive cells as a function of total nucleated cells may be readily obtained.

### B. Histology.

9

At various times post-transplantation, tissues are taken either by biopsy (of, e.g., the tissue growing beneath the left renal capsule) or by autopsy and frozen on O.C.T. for thin-section. Thereafter, sections are stained with the above monoclonal antibodies and counterstained with a secondary alkaline phosphatase-labelled secondary antibody. The presence of cells positive for the marker is then detected with the alkaline phosphatase reaction product of diaminobenzidine and visualized under light microscopy.

C. DNA analysis.

Cells taken by tail vein bleed or by resuspension of organ systems after biopsy or autopsy are treated by standard methods for the extraction of total cellular DNA. Total cellular DNA derived from SCID-hu mice has been analyzed using various probes and assay systems and compared in parallel to DNA obtained from untreated SCID mice or from normal human controls. The probes include the following: 1) Alu repeat (BLUR 8), a repetitive sequence of DNA found in the human genome but not homologous enough to murine sequences to cross-hybridize under the conditions used in these experiments, 2) human T-cell receptor (B chain) probes, 3) human Ig constant region probes, 4) human MHC Class I and Class II probes. For use in dot blots and in Southerns (run under standard conditions), these probes are first labelled with $^{32}$P by the hexamer-labelling technique. Rearrangements in the T- and/or B-cell receptor genes are sought by restriction endonuclease digestion of total cellular DNA, using known restriction maps and standard conditions. For polymerase chain reactions, using the thermostabile Taq 1 polymerase, a number of oligonucleotides have been constructed and/or obtained, including those to generate copies of the Alu repeat as well as of the Class I and Class II monomorphic sequences.

D. Karyotype analysis.

For karyotype analysis, cells taken by tail bleed have been put into culture with phytohemagglutinin to induce proliferation of human cells. At 48 hours, metaphase arrest is induced by the addition of colchicine and chromosomes prepared for analysis by light microscopy.

Results

1. Human fetal thymus grows in SCID mice.

The growth of human fetal thymus has been observed in most (>80%) SCID mice that have been transplanted with human fetal thymus (including >300 mice in successive experiments at various times over about an 18-month period). In those cases where tissue growth is not observed, problems related to implantation technique are likely to be the cause. The gross architecture is observed by re-opening the flank incision initially made to implant the thymic specimens and exposing the kidney. By 4-8 weeks post-implantation, marked increase in size is almost invariably evident (in very approximate terms ranging from a two-fold to a 20-30 fold increase). In several cases, fragments of human fetal thymus implanted into one mouse have been removed and transferred to a second, previously untreated mouse. In these cases as well, dramatic growth of thymic tissue has been subsequently observed. The thymus is similar to that of normal human fetal thymus in both color and in consistency. A well-defined vascular system may be observed with low-power magnification. When biopsy specimens are prepared for tissue section and stained with monoclonal antibodies against antigens present on human or murine cells, it is found that the cellular contents of the implanted thymus are mostly human (see below) and that the microscopic anatomy of the tissue has been generally conserved. Thus, well-defined medullary and cortical compartments are present, with characteristic staining of epithelial components with the antibodies MD1 and CDR2, respectively. Dendritic-like cells positive for human Class II determinants are found in the medulla and, to a lesser degree, in the cortex. Thymocytes which stain for the human T-cell markers CD3, CD4, CD8, and CD2 are also present. When analyzed by dual-laser FACS, the percentage of cells that are double-positive for CD4 and CD8, single-positive for CD4 or CD8, or double-negative for both markers is similar to that found in a normal, age-matched fetal thymus. The above findings have been made with mice that have received thymic tissue from human fetuses aged 9-22 gestational weeks.

The only difference noted to date between the SCID-hu thymus implant and a normal human fetal thymus is the presence, in the former but not in the latter, of dendritic-like cells in the cortex and (to a lesser degree) in the medulla which express murine Class II determinants. These cells are likely derived from murine, bone-marrow precursors which migrate into the vascularized SCID-hu thymus implant.

A number of important conclusions may be drawn from the above observations. First, human fetal thymus tissue is not rejected by the SCID mouse. Indeed, it is richly vascularized and grows instead. Secondly, by gross morphology and microscopic anatomy, the implanted human thymic tissue bears close resemblance to its normal human counterpart. Thus, the organ appears to maintain intrinsic properties for spatially correct regeneration.

Also, the number of and relative subset distribution of thymocytes appears to be normal. Finally, murine dendritic cells bearing murine Class II determinants are found in the SCID-hu thymus. These cells may be involved in "teaching" the developing human thymocytes to be tolerant of (and possibly MHC-restricted to) murine MHC antigens. Thereby, graft-versus-host disease may be averted.

2. Phenotypically mature human T-cells are found in the peripheral circulation of SCID-hu mice.

This phenomenon has been observed in >300 sets of experiments. By FACS analysis using monoclonal antibodies against human T-cell markers, it is predictably time-dependent, occurring 4-12 weeks after intravenous introduction of human fetal liver cells into a SCID mouse previously (or simultaneously) implanted with human fetal thymus.

Specifically, sets of mice received human fetal thymus implants. These sets were subdivided into groups: some received $10^7$ fetal liver cells (FLC) intravenously at the same time, some received $10^7$ fetal liver cells intrathymically two weeks later, some received no fetal liver cells at all. At three weeks, the peripheral blood of each was tested for the presence of cells staining for the human T-cell markers CD3, CD4, CD8, CD2; all were negative. By 4-12 weeks, however, such cells were visible on FACS (constituting 0.2-40% of the total mononucleated cell population). The ratio of $CD4^+$ to $CD8^+$ human T-cells in SCID-hu peripheral circulation during this time was between 3-4 (mean 3.5, n = 85, S.D. = 0.86), well within the normal range. Tested again between 12-15 weeks post-FLC transplantation, cells bearing these markers were no longer present. This general pattern was true irrespective of the route by which FLC cells were initially administered.

From this analysis it appears to take 4 weeks for significant numbers of cells bearing markers of human T-cells to appear in the peripheral blood of SCID-hu mice. Before that time, they are not present; after 12 weeks, they disappear. A corollary is that immunophenotyping is probably legitimate; that is, the antibodies that were used are only reactive in a time-dependent manner and appear not to bind indiscriminately to murine cells. Of interest, the transient wave of human T-cells in the periphery may occasionally be observed whether or not fetal liver cells are given. This appears to mean that cells that are resident within the fetal thymus, at the time that it is implanted, are also able to exit the thymus and to enter the peripheral circulation.

After it had been determined that peripheral blood cells bearing human markers were absent from the SCID-hu animals, all were once again reconstituted with FLC; notably, all of these animals retained the original human fetal thymus implants. In this second round of reconstitution, the animals were once again subdivided into groups: some received graded doses of FLC cells intravenously (ranging from $5\times10^6$ to $5\times10^7$); other received fragments of whole fetal liver, given both subcutaneously and intrasplenically. Analysis of peripheral blood 3 weeks later revealed the complete absence of cells bearing human markers. By 6 weeks, on the other hand, animals once again showed the presence of significant numbers of cells bearing the human markers CD3, CD4, CD8 and CD2 (approximately 4-30% of total mononuclear cells); notably 3-15% of these cells expressed the CD4 marker. When two mice were subjected to autopsy one week later (week 7), both showed cells in the periphery that stained either with OKT4 (a marker of the T helper cell lineage) or with OKT8 (a marker of the T cytotoxic/suppressor cell lineage(s)); the ratio of the two respective subpopulations was 2.3:1, well within the range of the normal ratio found in human peripheral blood itself. To ascertain whether these cells might actually home to the murine spleen, thymus, or lymph node, FACS analysis was also performed on cells obtained from these organs. In these cases, no cells bearing the human T-cell markers were observed. In subsequent FACS analyses of peripheral blood, all but one set of mice were observed to once again lose the peripheral population of human T-cells.

When SCID mice are implanted with whole fetal liver fragments and fetal thymus, and then given an intravenous injection of fetal liver cells, the same observations are made with a critical difference. Instead of lasting only 12 weeks, the human T-cells in the peripheral circulation are found to last up to 18 months (50% of animals show T-cells between 5 to 11 months). Again, the CD4/CD8 ratio is normal throughout.

This second round of reconstitution prompts a number of important conclusions. First, the phenomenon of a transient wave of human cells in the peripheral blood of SCID-hu mice given FLC intravenously is reproducible. Secondly, since the FLC were given intravenously and since they subsequently showed markers of mature T-cells, it is likely that they actually homed to and through the implanted human fetal thymus. Thirdly, the mature T-cells found in the periphery express a "physiologic" balance of CD4/CD8 subpopulations. Fourthly, it is apparent that these peripheral human T-cells do not actually home to the resident murine organs (spleen, lymph node, thymus). Thus, in ongoing experiments, the human equivalents have been implanted into SCID-hu mice. Finally, the introduction of whole fetal liver is found to generate a prolonged wave of human cells in the periphery. This observation implicates the presence of a self-renewing stem cell population within FLC, which is unable to replicate in the absence of fetal liver stromal cells.

Notwithstanding the apparent specificity of the monoclonal antibodies against human mature T-cell markers (note, even in those mice that showed high percentages of staining cells in the peripheral blood, spleen cell populations analyzed at the same time were negative), it remained a formal possibility that the staining was due to an unforeseen artifact. To control for this possibility, DNA analysis of the cells found in the peripheral blood was performed using a probe specific for human, but not murine, repetitive DNA sequences (the ALU repeat: BLUR-8). By this analysis, it was found that the peripheral blood cells derived from the SCID-hu mouse not only bear the phenotypic markers characteristic of human T-cells, but also contain DNA that strongly hybridizes with the human ALU probe.

When SCID mice are implanted with human fetal thymus and lymph node, and then given an intravenous injection of human fetal liver cells, human IgG may also be detected in the serum. The amount of IgG varies between 5-10% of that found normally. The plasma cells producing the IgG can be localized to the implanted human lymph node. Human IgG production requires at least the introduction of human lymph node into SCID mice.

In a specific experiment SCID mice were maintained in bonneted isolation cages and received TMS in suspension through the drinking water for 3 days of each week (40 mg of trimethoprim and 200 mg of sulfamethoxazole per 5 ml of suspension; 0.125 ml of suspension per 4 ml of drinking water per mouse per day). The drinking water bottle was turned daily while the drug was being administered; standard drinking water was substituted for the remaining 4 days of each week. A 9 g.w. human fetal thymus lobe 0.5 by 0.5 by 2 mm in size and 0.5 by 0.5 by 2 mm segment of a human lymph node were implanted under the left kidney capsule. These organs were obtained from an HLA-A2$^+$, HLA-B7$^-$ donor. Simultaneously, the mouse had received $10^7$ liver cells intravenously; these cells, in contrast, were HLA-A2$^-$, HLA-B7$^+$.

The fetal liver cells were obtained by mincing and cells remaining suspended in RPMI 1640 medium with 10% FCS were separated into mononuclear fraction by ficoll-hypaque centrifugation; the interface cells were then washed 3x in RPMI 1640 with 10% FCS and resuspended at a concentration of $10^8$ cells/ml for intravenous administration.

For implantation of tissue, mice were first anesthetized with halothane. A 1-cm flank incision was made to expose the left kidney. Sutures were placed to approximate successive peritoneal and fascial layers and metal clips were secured over the wound to ensure healing. Suspended fetal liver cells were injected intravenously by a retro-orbital approach with a 30-gauge needle.

Frozen serial fixed sections of the thymus four weeks after implantation were compared to a normal age-matched fetal thymus. The microscopic anatomy was found to be comparable, with the exception of murine Class II-positive cells having a dendritic morphology being present. In particular, all of the thymocytes were HLA-A2$^-$, HLA-B7$^+$; therefore, they were derived from stem cells within the FLC preparation given intravenously.

When peripheral blood from the SCID-hu mice were analyzed, human T-cells with a CD4/CD8 ratio of 3.5 were found between weeks 4-12. These cells expressed the antigen HLA-B7, but not HLA-A2; therefore, they were fully differentiated cells from the original FLC source, having been processed first through the HLA-A2$^+$, HLA-B7$^-$ thymus.

To better determine which of the various FLC subpopulations actually give rise to the mature human T-cells (e.g., which subpopulation(s) possessed stem cell activity), FLC from an HLA-A2$^-$, HLA-B7$^+$ donor were depleted of CD4$^+$, CD8$^+$, M1$^+$, M3$^+$, Ig$^+$, A$^+$/B$^+$ cells (removing T, B, myelomonocytic, erythrocytic, and granulocytic cells ) to produce a "lineage-marker minus" (Lin$^-$) population. This population was enriched by FACS into two subpopulations with an antibody against human Thy-I: Lin$^-$ Thy-I$^-$ and Lin$^-$ Thy-I$^+$. These populations were then introduced intravenously into SCID mice with a HLA-A2$^+$, HLA-B7$^-$ human thymus implant. The Lin$^-$ Thy-I$^+$ subpopulation was found to give rise to HLA-B7$^+$ T-cells later; the Lin$^-$ Thy-I$^-$ population was inactive. Therefore, the Lin$^-$ Thy-I$^+$ population is likely to be inclusive of human hematopoietic stem cell activity.

Finally, when serum specimens from the SCID-hu mice were analyzed, they were found to contain human IgG at levels of 1-10 mg/ml. Human lymph node biopsies showed the presence of human IgG-containing plasma cells by immunohistochemical stain.

3. Infection with CMV.

A. SCID-hu mice implanted with thymus and liver in the kidney capsule were inoculated in the graft with fresh clinical isolates of human CMV, strains Toledo (given at $5\times10^6$ PFU) and DK (given at $7\times10^5$) where Toledo was isolated from a diseased fetus (cytomegalic inclusion disease) and DK was isolated from an adult AIDS patient. Both of these strains grew in the implant and at 2, 5, 9 and 15 days post inoculation, two animals were sacrificed and the tissue removed, minced, sonicated and titered for the presence of human CMV by plaque assay on human fibroblasts. For both strains, peaks were observed at about $2 \times 10^5$ PFU/ml at 9 days for Toledo and at about $2 \times 10^4$ PFU/ml at 5 days for DK, with the Toledo titer diminishing to about $10^5$ at day 15, while the DK dropped to about 10 PFU/ml by day 15. The test was repeated with 3-4 animals for each determination except for 15 days, which was 2 animals.

| THYMUS/LIVER IMPLANT* | |
| --- | --- |
| Days Post Infection | PFU/Tissue Geometric Mean $\times10^{-3}$** |
| 2 | 0.01 |
| 5 | 668 |
| 12 | 3154 |
| 15 | 812 |
| 35 | 195 |

\* Toledo strain at $1$-$2\times10^6$ PFU injected.
\*\* Average of 2 wells per value. 3-9 mice per determination.

B. Fetal skin was implanted on the back in the CB-17 scid/scid mouse substantially as described. After 24-40 weeks post implant, the skin engrafted mice were inoculated intragraft and intravenously with fresh clinical isolates of human CMV, strains Toledo (given at $5 \times 10^6$ PFU) and DK (given at $7 \times 10^5$) and the tissue assayed at 2, 5, 9 and 15 days post inoculation. In skin, both viruses grew with similar qualitative and quantitative characteristics, both peaking at about 5 days at about $6 \times 10^3$ PFU/ml and diminishing to fewer than about 10 PFU/ml. Particular attention was given to removing the fatty underlayer of mouse tissue adhering to the human skin graft.

C. In the absence of human tissue capable of maintaining CMV replication, intravenous injection of Toledo ($5 \times 10^6$) and DK ($7 \times 10^5$) resulted in the substantial absence of CMV in the peripheral blood within 2 to 4 hours post inoculation.

D. Approximately 10-20 $\mu$l of CMV Toledo passage 9 was injected into bone, where the bone had been implanted as described subsequently in mice which had not been irradiated. A total of 16 mice were infected with 4 mice sacrificed on days 2, 5, 9 and 12-14, while 4 mice were used as negative controls, which were sacrificed on days 2 and 12-14. The bone size was about average.

The bone was surgically removed from the mouse, minced and placed in 2.2 ml DME/50% FCS and sonicated for 10 sec at a setting of 4-5. Between each of the 3 rounds of sonication, tubes were cooled on ice for about 15 sec. The sonicate was diluted and titered on fibroblast plates in duplicate at each dilution. 1 ml of each dilution was incubated 1-2 hrs on fibroblast plates at 37°C. The sonicate was removed and replaced by 2 ml DME/10% FCS complete. 2-3 days later, 1 ml media was added. Fibroblasts were cultured one week and then fixed with methanol, followed by staining with Geimsa. Colonies (PFU) were counted.

About half of the total dispersed cells from minced bone (about $5\times10^5$ cells) was assayed in the methylcellulose assay with the other half being used for sonication along with the remaining bone debris.

The following table indicates the results obtained using the plaque assay described above.

| BONE IMPLANT | |
|---|---|
| Days Post Infection | PFU/Tissue Geometric Mean[*] |
| 2 | 17 |
| 5 | 2558 |
| 9 | 965 |
| 13 | 68 |
| 29 | 25 |

[*] Average of 2 wells per value.

In the next study, the thymus and liver implant was employed for the CMV infection, but ganciclovir was provided at varying concentrations in the drinking water. Mice were studied where the mice were maintained on the drug for two weeks, beginning 6 h after infection and the level of CMV infection determined at the end of the 2-week period. In the next study, the mice were maintained for two weeks on the drug, then two weeks off the drug, and the level of viremia determined at the end of the 4-week period.

The following tables indicate the results.

| THYMUS/LIVER IMPLANT WITH GANCICLOVIR | |
|---|---|
| Ganciclovir $\mu$l/ml | PFU/Tissue Geometric Mean $x10^{-3*}$ |
| 0 | 538 |
| 100 | 124 |
| 500 | 22 |
| 1500 | 0.3 |

[*] Average of 2 wells per value, 4 mice. Results determined after 2 weeks on ganciclovir.

| THYMUS/LIVER IMPLANT WITH GANCICLOVIR AND VIRUS REBOUND | |
|---|---|
| Ganciclovir $\mu$l/ml | PFU/Tissue Geometric Mean $x10^{-3*}$ |
| 0 | 659[*] |
| 500 | 487[*] |
| 1500 | 45[**] |

[*] Determination from 3 values from 3 animals.
[**] Determination from 1 value from 1 animal.

E. The next study employed colon as the fetal tissue (see implantation of gut, *supra*). Following the procedure described above, the colon was injected with 10-20 $\mu$l of CMV Toledo Passage 9 (~ 1-2 x 10$^6$) and the course of the infection followed at days 2, 5, 9, and 12-14. Following the procedure described above, the plaque-forming units were determined 2 weeks after infection.

The following table indicates the results.

| COLON IMPLANT | |
|---|---|
| Days Post Infection | PFU/Tissue Geometric Mean |
| 2 | -- |
| 5 | 484 |
| 8 | 177 |
| 12 | -- |

14

5. Implantation of various tissues and organs.

A. Implantation of Bone.

Human fetal long bones (17-22 g.w.) of about 1 cm in length (1-2 bones) were transplanted subcutaneously or intraperitoneally into CB-17 scid/scid mice which were preconditioned with 200-300 rads whole body irradiation or were untreated. At different time points after transplantation, bones were taken out and cells recovered from them were stained with either human specific antibody, MEM-43, or mouse specific antibody, Ly.5.1 and analyzed by FACS or cytospin preparations. Sections from the transplants were prepared for routine histology.

Human hematopoiesis was not observed by histology or by cytospin preparations at 2-3 weeks after transplantation. The majority of cells recovered from human bone grafts were positive for MEM-43. Scatter analysis by flow cytometry did not show lymphoid or myeloid populations, suggesting that the majority of cells were non-hematopoietic in origin.

At 4-5 weeks after transplantation, signs of hematopoiesis (i.e., presence of blast cells, immature forms of myelomonocytic cells and erythroblasts) were observed in cytospin preparations in most cases analyzed. The MEM-43 positive cells in these samples showed a scatter profile similar to that of the fetal bone marrow samples. Cells of the myelomonocytic lineage, B cell lineage and the erythroid lineage were shown by immunofluorescent staining with LeuM1 (CD15), CD10 and CD19, and anti-human glycophorin A, respectively. Animals preconditioned with irradiation (200-300 rads) show a significant number of circulating myeloid (CD33) or B lymphoid (CD 19, Ig$^+$) cells in the peripheral blood (0.5-30%).

B. Implantation of Skin.

The skin from (1) fetus (16-22 g.w., thigh, scalp and plantar), (2) neonatal skin (foreskin), and (3) adult (trunk) was grown on the back of SCID mice. The subcutaneous tissues of the skin for implantation were removed as much as possible, put as a whole in the mouse skin and stitched. After implantation (2-3 weeks), crust formation on the implanted skin was observed, followed by growth of the implanted skin. Histological examination revealed a very similar structure to normal human skin, i.e., epithelial cells in the epidermis, hair follicles, and adnexal glands and dermal structure.

C. Implantation of Placenta.

Small pieces of placental tissue with chorionic villi (16-18 g.w.) were implanted under the kidney capsule of the SCID mouse, as described previously. The growth of placental tissue with cytotrophoblasts was observed 4 weeks after implantation.

D. Implantation of Gut.

Small intestine including appendix from the fetus (16-18 g.w.) was cut into pieces (2 x 2 x 3 mm) in cross-section with or without mesenteric tissue, and implanted under the kidney capsule of the SCID mouse. These grafts made cystic structures which contain mucinous fluid inside. The wall of the cyst was lined with the mucosal layer, accompanied with smooth muscle layers similar to those observed in the normal gut.

E. Implantation of Lung.

Small pieces (2 x 2 x 2 mm) of fetal lung (18-22 g.w.) were implanted into the SCID mouse (1) under the kidney capsule, (2) in the fourth mammary fat pad, or (3) intraperitoneally. In all cases, rapid growth of implanted lung could be observed. A structure similar to that of fetal lung, including alveolar structure, bronchi and bronchioles with epithelial cells, smooth muscles and soft bones was observed in the grafts 4 weeks after implantation.

F. Implantation of Pituitary.

Fetal pituitary gland (18-22 g.w.) was cut into small pieces and implanted under the kidney capsule of the SCID mouse. After implantation (5-6 weeks), the structure of the implant was very similar to that of the anterior lobe of the pituitary gland with the acinar structure composed of chromophobic, acidophilic and basophilic cells.

G. Implantation of Pancreas.

Fetal pancreatic tissues (18-22 g.w.) were cut into small pieces and implanted under the kidney capsule of the SCID mouse. Pancreatic grafts grew well under the kidney capsule. Histological examination of the grafts (4-6 weeks after implantation) revealed that all the cellular components of pancreatic tissue including acinar glands, ducts with varying size, and well grown islets were of analogous structure to that of normal pancreas. In come cases, a slight fibrotic change between acinus was observed, similar to chronic pancreatitis although the infiltration of mononuclear cells did not accompany the fibrotic change.

It is evident from the above, that by introducing xenogeneic tissue into an immunocompromised host, where the xenogeneic tissue is able to remain viable and functional, a wide variety of advantageous results ensue. One can provide for the testing of efficacy and influence on the immune system of a wide variety of agents, which may include drugs, therapies, pathogens, or the like. These agents may be evaluated for efficacy against diseases, where the xenogeneic tissue may be introduced into the host and infected with the pathogen, followed by determining the effect of an agent on the progress of the pathogen in the xenogeneic tissue or external to the xenogeneic tissue.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

**Claims**

1. A method for determining the response of a pathogen other than a member of the species retroviridae to a stimulus, wherein said pathogen is tropic to a species, said method comprising:

   administering a stimulus capable of inducing a physiological response from cells comprising said tissue or pathogen infecting said tissue to an immunocompromised host other than a primate lacking at least functional T cells and comprising vascularized non-transformed solid organ tissue of said species, said stimulus being administered in an amount to produce said physiological response;

   whereby the effect of said stimulus is determined.

2. A method according to claim 1, wherein said solid organ tissue is hematopoietic tissue.

3. A method according to claim 1, wherein said host is murine.

4. A method according to claim 3, wherein said murine host is scid/scid.

5. A method for determining the response of human cytomegalovirus to a drug, said method comprising: administering said drug to an immunocompromised host comprising at least one fetal human tissue of the group a combination of thymus and liver, mucosal tissue or bone marrow infected with human cytomegalovirus, wherein said tissue is present in said immunocompromised host which is other than a primate and lacking at least functional T cells and comprising said tissue vascularized and non-transformed; and

   determining the effect of said drug on the proliferation of said cytomegalovirus.

6. A SCID/hu mouse infected in human tissue with a pathogen other than a member of the retroviridae family having a tropism for human tissue.

7. A SCID/hu mouse according to claim 6, wherein said tissue is a combination of fetal thymus and liver.

8. A SCID/hu mouse according to claim 6, wherein said tissue is mucosal tissue.

16

9. A SCID/hu mouse according to claim 6, wherein said tissue is bone marrow.

10. A SCID/hu mouse according to claim 6, wherein said pathogen is cytomegalovirus.

**PARTIAL EUROPEAN SEARCH REPORT**
which under Rule 45 of the European Patent Convention
shall be considered, for the purposes of subsequent
proceedings, as the European search report

Application number

| DOCUMENTS CONSIDERED TO BE RELEVANT | | | EP 91113061.5 |
|---|---|---|---|
| Category | Citation of document with indication, where appropriate, of relevant passages | Relevant to claim | CLASSIFICATION OF THE APPLICATION (Int. Cl.⁵) |
| | No documents have been disclosed (see supplemental sheet B) ---- | | C 12 Q 1/18<br>C 12 Q 1/70<br>A 61 K 35/12<br>G 01 N 33/569 |
| | | | TECHNICAL FIELDS SEARCHED (Int. Cl.⁵) |

**INCOMPLETE SEARCH**

The Search Division considers that the present European patent application does not comply with
the provisions of the European Patent Convention to such an extent that it is not possible to carry
out a meaningful search into the state of the art on the basis of some of the claims.

Claims searched completely: –
Claims searched incompletely: –
Claims not searched: 1-5,6-10
Reason for the limitation of the search:

1-5 Art. 52(4) EPC

6-10 Art. 53(b) EPC

| Place of search | Date of completion of the search | Examiner |
|---|---|---|
| VIENNA | 13-11-1991 | SCHNASS |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO Form 1505.1. 03.82